# EUROPEAN PATENT SPECIFICATION

(11) **EP 1 335 730 B1**
(45) Date of publication and mention of the grant of the patent: **01.06.2005**
(21) Application number: 01983728.5
(22) Date of filing: 19.11.2001
(51) Int. Cl.: A61K 31/505, A61P 15/00

(54) **USE OF PDE5 INHIBITORS FOR THE TREATMENT OF PREMATURE EJACULATION**
VERWENDUNG VON PDE5 INHIBITOREN ZUR BEHANDLUNG VORZEITIGER EJAKULATION
UTILISATION DES INHIBITEURS DE PDE5 POUR LE TRAITEMENT DE L'EJACULATION PRECOCE

(30) Priority: 20.11.2000 GB 0028245
(43) Date of publication of application: 20.08.2003
(73) Proprietor: Pfizer Limited, Sandwich, Kent CT13 9NJ (GB); Pfizer Inc., New York, New York 10017 (US)
(72) Inventor: BOOLELL, Mitradev, Sandwich, Kent CT13 9NJ (GB)
(74) Representative: Rutt, Jason Edward
(86) International application number: PCT/IB2001/002180
(87) International publication number: WO 2002/040027

(56) References cited:
- MEINHARDT W ET AL: "COMPARATIVE TOLERABILITY AND EFFICACY OF TREATMENTS FOR IMPOTENCE" DRUG SAFETY, ADIS PRESS, AUCKLAND, NZ, vol. 20, no. 2, 1999, pages 133-146, XP000853834 ISSN: 0114-5916
- KULKARNI S K ET AL: "PHARMACOTHERAPY OF MALE ERECTILE DYSFUNCTION WITH SILDENAFIL" INDIAN JOURNAL OF PHARMACOLOGY, XX, XX, vol. 30, no. 6, 1998, pages 367-378, XP000921330 ISSN: 0253-7613

## Description

This invention relates to the use of cyclic guanosine 3', 5'-monophosphate phosphodiesterase type five inhibitors (hereinafter PDE5 inhibitors) for the treatment of premature ejaculation (PE). Particular PDE5 inhibitors are sildenafil, IC-351, vardenafil, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one and 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one.

According to the specification of our International patent application WO94/28902 we have discovered that compounds which are inhibitors of the cGMP PDE5 enzyme are potent and effective compounds for the treatment of male erectile dysfunction (MED, impotence) and for female sexual disorders. This discovery led to the development of the compound sildenafil (5-[2-ethoxy-5-(4-methyl-1-piperazinylsulphonyl)phenyl]-1-methyl-3-n-propyl-1,6-dihydro-7H-pyrazolo[4,3-d]pyrimidin-7-one) (VIAGRA™) which has proved to be outstandingly successful as the first orally effective treatment for MED.

PE is a relatively common sexual dysfunction in men. It has been defined in several different ways but the most widely accepted is the Diagnostic and Statistical Manual of Mental Disorders IV one which states: "PE is a lifelong persistent or recurrent ejaculation with minimal sexual stimulation before, upon or shortly after penetration and before the patient wishes it. The clinician must take into account factors that affect duration of the excitement phase, such as age, novelty of the sexual partner or stimulation, and frequency of sexual activity. The disturbance causes marked distress or interpersonal difficulty."

The International Classification of Diseases 10 definition states: "There is an inability to delay ejaculation sufficiently to enjoy lovemaking, manifest as either of the following: (1) occurrence of ejaculation before or very soon after the beginning of intercourse (if a time limit is required: before or within 15 seconds of the beginning of intercourse); (2) ejaculation occurs in the absence of sufficient erection to make intercourse possible. The problem is not the result of prolonged abstinence from sexual activity."

Other definitions that have been used include classification on the following criteria:
- Related to the partner's orgasm
- Duration between penetration and ejaculation
- Number of thrusts and capacity for voluntary control

Psychological factors may be involved in PE, with relationship problems, anxiety, depression, prior sexual failure all playing a role.

The estimated prevalence of PE is about 22-38% of the male population; unlike MED it has no definite correlation with age. Taking an average prevalence of 30%, that would make an estimated 24 million sufferers in the US (males aged 18-65 was 80 million in 1995). There is little data on prevalence by severity. It is estimated that the operational definition of PE may apply to 5-10% of men, however, less than 0.2% present for treatment. The availability of an orally effective therapy is very likely to alter this situation.

Urologists currently form the bulk (59%) of physicians treating PE; GPs form 33% of doctors treating the condition. Sex therapists, behavioural therapists and counsellors also treat patients with PE. Experts estimate that 50% of presenters do so because of the impact the condition has on the relationship with the partner. Stress, relationship difficulties and/or effect on quality of life are the key triggers for sufferers to seek treatment for PE.

Ejaculation is dependent on the sympathetic and parasympathetic nervous systems. Efferent impulses via the sympathetic nervous system to the vas deferens and the epididymis produce smooth muscle contraction, moving sperm into the posterior urethra. Similar contractions of the seminal vesicles, prostatic glands and the bulbourethral glands increase the volume and fluid content of semen. Expulsion of semen is mediated by efferent impulses originating from the nucleus of Onuf in the spinal cord, which pass via the parasympathetic nervous system and cause rhythmic contractions of the bulbocavernous, ischiocavernous and pelvic floor muscles. Cortical control of ejaculation is still under debate in humans. In the rat the medial pre-optic area and the paraventricular nucleus of the hypothalamus seem to be involved in ejaculation.

There are at present no approved drugs available for treating PE. The most commonly off-label prescribed medications are the anti-depressants (for example clomipramine) and the selective seretonin re-uptake inhibitors (for example paroxetine and sertraline). These drugs are often not well accepted by patients because they are regarded as anti-depressants. They are used 'off-label', and though effective when used as required (i.e. 'prn'), due to their long pharmacokinetic Tₘₐₓ (time to maximum drug concentration in plasma following oral administration of the drug) they are likely to have a slow onset of action. Side-effects common to this class of drugs can be seen when used chronically. Behavioural therapy has been the other management tool but has not been very efficacious and has a high drop-out and relapse rate. New, more effective therapies, are required.

According to a first aspect, the invention provides the use of a PDE5 inhibitor in the manufacture of a medicament for treating premature ejaculation in patients with normal erectile function.

By PDE5 inhibitors it is meant a compound which is a potent and selective inhibitor of the cGMP PDE5 isoenzyme.

In accordance with the invention, patients with normal erectile function are those who are capable of achieving an erection (without any medicament or medical device such as a vacuum pump) sufficient for vaginal penetration and are able to maintain the erection until ejaculation. PE in these patients is typically primary PE.

In accordance with the invention, patients suffering from PE but with normal erectile function experience persistent or recurrent ejaculation with minimal sexual stimulation shortly after penetration and before the person wishes it. In addition, the PE in these patients is not situational or secondary to a known organic cause. Typically PE in these patients has been present since their first sexual experience.

In a preferred embodiment of the invention, patients with normal erectile function are those who attain a score of more than 22 (preferably more than 25) on the Erectile Function Domain Questionnaire (see hereinafter).

Hereinafter the term "the PDE5 inhibitor" means the PDE5 inhibitors for use with the invention. The term includes pharmaceutically acceptable salts, solvates and polymorphs of the PDE5 inhibitors for use with the invention.

The suitability of the PDE5 inhibitor can be readily determined by evaluation of its potency and selectivity using literature methods followed by evaluation of its toxicity, absorption, metabolism, pharmacokinetics etc, in accordance with standard pharmaceutical practice.

Preferably, the PDE5 inhibitors have an IC50 against the PDE5 enzyme of less than 100 nanomolar, more preferably, at less than 50 nanomolar.

IC50 values for the PDE5 inhibitors may be determined using the PDE5 assay in the Test Methods Section hereinafter.

Preferably the PDE5 inhibitors are selective for the PDE5 enzyme. Preferably they have a selectivity for PDE5 over PDE3 of greater than 100 fold, more preferably greater than 300 fold. More preferably the PDE5 inhibitors have a selectivity over both PDE3 and PDE4 of greater than 100 fold, more preferably greater than 300 fold.

Selectivity ratios may readily be determined by the skilled person, by ratio of corresponding IC50 values for the particular enzymes concerned. IC50 values for the PDE3 and PDE4 enzyme may be determined using established literature methodology, see S A Ballard et al, Journal of Urology, 1998, vol. 159, pages 2164-2171.

Preferably the PDE5 inhibitors have an IC50 against PDE5 of less than 100 nM and a selectivity over PDE3 of greater than 100 fold.

Examples of PDE5 inhibitors for use with the invention are:

The pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the compounds disclosed in published international application WO95/19978; the compounds disclosed in published international application WO 99/24433 and the compounds disclosed in published international application WO 93/07124.

The pyrazolo [4,3-d]pyrimidin-7-ones disclosed in published international application WO 01/27112; the pyrazolo [4,3-d]pyrimidin-7-ones disclosed in published international application WO 01/27113; the compounds disclosed in EP-A-1092718 and the compounds disclosed in EP-A-1092719.

Preferred PDE5 inhibitors for use with the invention:
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);
5-(2-ethoxy-5-morpholinoacetylphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see WO 01/27113, Example 8);
5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see WO 01/27113, Example 15);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO 01/27113, Example 66);
5-(5-Acetyl-2-propoxy-3-pyridinyl)-3-ethyl-2-(1-isopropyl-3-azetidinyl)-2,6-dihydro-7H-pyrazolo[4,3-*d*]pyrimidin-7-one (see WO 01/27112, Example 124);
5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one (see WO 01/27112, Example 132);
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (IC-351), i.e. the compound of examples 78 and 95 of published international application WO95/19978, as well as the compound of examples 1, 3, 7 and 8;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e. the compound of examples 20, 19, 337 and 336 of published international application WO99/24433; and
the compound of example 11 of published international application WO93/07124 (EISAI); and
compounds 3 and 14 from Rotella D P, *J. Med. Chem.,* 2000, 43, 1257.

Still further PDE5 inhibitors for use with the invention include: 4-bromo-5-(pyridylmethylamino)-6-[3-(4-chlorophenyl)-propoxy]-3(2H)pyridazinone; 1-[4-[(1,3-benzodioxol-5-ylmethyl)amiono]-6-chloro-2-quinozolinyl]-4-piperidinecarboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3-(2H)pyridazinone; 1-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)arnino]-6-chloro-2- quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer) and Sch-51866.

More preferred PDE5 inhibitors for use with the invention are selected from the group:
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil);
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (IC-351);
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; and
5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one and pharmaceutically acceptable salts thereof.

A particularly preferred PDE5 inhibitor is 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) (also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine) and pharmaceutically acceptable salts thereof. Sildenafil citrate is a preferred salt.

Oral bioavailablity refers to the proportion of an orally administered drug that reaches the systemic circulation. The factors that determine oral bioavailability of a drug are dissolution, membrane permeability and metabolic stability. Typically, a screening cascade of firstly in vitro and then in vivo techniques is used to determine oral bioavailablity.

Dissolution, the solubilisation of the drug by the aqueous contents of the gastrointestinal tract (GIT), can be predicted from in vitro solubility experiments conducted at appropriate pH to mimic the GIT. Preferably the PDE5 inhibitors have a minimum solubility of 50 mcglml. Solubility can be determined by standard procedures known in the art such as described in Adv. Drug Deliv. Rev. 23, 3-25, 1997.

Membrane permeability refers to the passage of a compound through the cells of the GIT. Lipophilicity is a key property in predicting this and is defined by *in vitro* Log D_{7.4} measurements using organic solvents and buffer. Preferably the PDE5 inhibitors have a Log D_{7.4} of -2 to +4, more preferably -1 to +3. The log D can be determined by standard procedures known in the art such as described in J. Pharm. Pharmacol. 1990, 42:144.

Cell monolayer assays such as CaCo₂ add substantially to prediction of favourable membrane permeability in the presence of efflux transporters such as p-glycoprotein, so-called caco-2 flux. Preferably, the PDE5 inhibitors have a caco-2 flux of greater than 2x10⁻⁶cms⁻¹, more preferably greater than 5x10⁻⁶cms⁻¹.

The caco flux value can be determined by standard procedures known in the art such as described in J. Pharm. Sci, 1990, 79, 595-600.

Metabolic stability addresses the ability of the GIT or the liver to metabolise compounds during the absorption process: the first pass effect. Assay systems such as microsomes, hepatocytes etc are predictive of metabolic liability. Preferably the PDE5 inhibitors show metabolic stability in the assay system that is commensurate with a hepatic extraction of less then 0.5. Examples of assay systems and data manipulation are described in Curr. Opin. Drug Disc. Devel., 201, 4, 36-44, Drug Met. Disp.,2000, 28, 1518-1523.

Because of the interplay of the above processes further support that a drug will be orally bioavailable in humans can be gained by *in vivo* experiments in animals. Absolute bioavailability is determined in these studies by administering the compound separately or in mixtures by the oral route. For absolute determinations (% absorbed) the intravenous route is also employed. Examples of the assessment of oral bioavailability in animals can be found in Drug Met. Disp.,2001, 29, 82-87; J. Med Chem , 1997, 40, 827-829, Drug Met. Disp.,1999, 27, 221-226.

The PDE5 inhibitors can be administered alone but will generally be administered in admixture with a suitable pharmaceutical excipient, diluent or carrier selected with regard to the intended route of administration and standard pharmaceutical practice.

For example, the PDE5 inhibitors can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The PDE5 inhibitors may also be administered as fast-dispersing or fast-dissolving dosage forms or in the form of a high energy dispersion or as coated particles. Suitable formulations may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

The following formulation examples are illustrative only and are not intended to limit the scope of the invention. Active ingredient means a PDE5 inhibitor.

### Formulation 1:

A tablet is prepared using the following ingredients :
Active ingredient (50 mg) is blended with cellulose (microcrystalline), silicon dioxide, stearic acid (fumed) and the mixture is compressed to form tablets.

### Formulation 2 :

An intravenous formulation may be prepared by combining active ingredient (100 mg) with isotonic saline (1000 ml).

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the PDE5 inhibitors may be combined with various sweetening or flavouring agents, colouring matter or dyes, with emulsifying and/or suspending agents and with diluents such as water, ethanol, propylene glycol and glycerin, and combinations thereof.

Modified release and pulsatile release dosage forms may contain excipients such as those detailed for immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating.

Fast dispersing or dissolving dosage formulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used i.e. where the drug substance is insoluble a fast dispersing dosage form can be prepared and where the drug substance is soluble a fast dissolving dosage form can be prepared.

The PDE5 inhibitors can also be administered parenterally, for example, intracavernouslly, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The following dosage levels and other dosage levels herein are for the average human subject having a weight range of about 65 to 70 kg. The skilled person will readily be able to determine the dosage levels required for a subject whose weight falls outside this range, such as children and the elderly.

The dosage of the PDE5 inhibitor in such formulations will depend on its potency, but can be expected to be in the range of from 1 to 500 mg for administration up to three times a day. In the case of sildenafil, a preferred dose is in the range 10 to 100 mg (e.g. 10, 25, 50 and 100 mg) which can be administered once, twice or three times a day (preferably once). However the precise dose will be as determined by the prescribing physician and will depend on the age and weight of the patient and severity of the symptoms.

For oral and parenteral administration to human patients, the daily dosage level of the PDE5 inhibitors will usually be from to 5 to 500 mg/kg (in single or divided doses).

Thus tablets or capsules of the PDE5 inhibitors may contain from 5 mg to 250 mg (preferably 10 to 100 mg) of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that the PDE5 inhibitors may be taken as a single dose as needed or desired (i.e. prn). It is to be appreciated that all references herein to treatment include acute treatment (taken as required) and chronic treatment (longer term continuous treatment).

The PDE5 inhibitors can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the PDE5 inhibitor and a suitable powder base such as lactose or starch.

Aerosol or dry powder formulations are preferably arranged so that each metered dose or "puff" contains from 1 µg to 50 mg of a PDE5 inhibitor for delivery to the patient. The overall daily dose with an aerosol will be in the range of from 1µg to 50 mg which may be administered in a single dose or, more usually, in divided doses throughout the day.

Alternatively, the PDE5 inhibitors can be administered in the form of a suppository or pessary, or they may be applied topically in the form of a gel, hydrogel, lotion, solution, cream, ointment or dusting powder. The PDE5 inhibitors may also be dermally or transdermally administered, for example, by the use of a skin patch. They may also be administered by the pulmonary or rectal routes.

For application topically to the skin, the PDE5 inhibitors can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended
or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The PDE5 inhibitors may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

Generally, in humans, oral administration of the PDE5 inhibitors is the preferred route, being the most convenient. In circumstances where the recipient suffers from a swallowing disorder or from impairment of drug absorption after oral administration, the drug may be administered parenterally, sublingually or buccally.

A further preferred route is topically via the skin, preferably locally to the male genitalia.

In an embodiment of the invention, the PDE5 inhibitors may also be combined with one or more additional active agents for treating PE in patients with normal erectile function, the active agent being selected from the following list:
1) one or more naturally occurring or synthetic prostaglandins or esters thereof; suitable prostaglandins for use herein include compounds such as alprostadil, prostaglandin E₁, prostaglandin E₀, 13, 14 - dihydroprosta glandin E₁, prostaglandin E₂, eprostinol, natural synthetic and semisynthetic prostaglandins and derivatives thereof including those described in WO-00033825 and/or US 6,037,346 issued on 14th March 2000 all incorporated herein by reference, PGE₀, PGE₁, PGA₁, PGB₁, PGF₁ α, 19-hydroxy PGA₁, 19-hydroxy - PGB₁, PGE₂, PGB₂, 19-hydroxy-PGA₂, 19-hydroxy-PGB₂, PGE₃α, carboprost tromethamine dinoprost, tromethamine, dinoprostone, lipo prost, gemeprost, metenoprost, sulprostune, tiaprost and moxisylate;
2) one or more α-adrenergic receptor antagonists (also known as α-adrenoceptor blockers, α-receptor blockers or α-blockers); suitable α₁-adrenergic receptor antagonists include: phentolamine, prazosin, phentolamine mesylate, trazodone, alfuzosin, indoramin, naftopidil, tamsulosin, phenoxybenzamine, rauwolfa alkaloids, Recordati 15/2739, SNAP 1069, SNAP 5089, RS17053, SL 89.0591, doxazosin, terazosin and abanoquil; suitable α₂- adrenergic receptor antagonists include dibenarnine, tolazoline, trimazosin, efaroxan, yohimbine, idazoxan clonidine and dibenarnine; suitable non-selective α-adrenergic receptor antagonists include dapiprazole; further α- adrenergic receptor antagonists are described in PCT application WO99/30697 published on 14th June 1998 and US patents: 4,188,390; 4,026,894; 3,511,836; 4,315,007; 3,527,761; 3,997,666; 2,503,059; 4,703,063; 3,381,009; 4,252,721 and 2,599,000 each of which is incorporated herein by reference;
3) one or more vasodilator agents; suitable vasodilator agents for use herein include nimodepine, pinacidil, cyclandelate, isoxsuprine, chloroprumazine, halo peridol, Rec 15/2739 and trazodone;
4) one or more ergot alkoloids; suitable ergot alkaloids are described in US patent 6,037,346 issued on 14th March 2000 and include acetergamine, brazergoline, bromerguride, cianergoline, delorgotrile, disulergine, ergonovine maleate, ergotamine tartrate, etisulergine, lergotrile, lysergide, mesulergine, metergoline, metergotamine, nicergoline, pergolide, propisergide, proterguride, terguride;
5) one or more angiotensin receptor antagonists such as losartan;
6) one or more calcium channel blockers such as amlodipine;
7) one or more antagonists of endothelin receptors and inhibitors or endothelin-converting enzyme;
8) one or more cholesterol lowering agents such as statins (e.g. atorvastatin/Lipitor- trade mark) and fibrates;
9) one or more acetylcholinesterase inhibitors such as donezipil;
10) one or more estrogen receptor modulators and/or estrogen agonists and/or estrogen antagonists, preferably raloxifene or lasofoxifene, (-)-cis-6-phenyl-5-[4-(2-pyrrolidin-1-yl-ethoxy)-phenyl]-5,6,7,8-tetrahydronaphthalene-2-ol and pharmaceutically acceptable salts thereof the preparation of which is detailed in WO 96/21656;
11) one or more further PDE inhibitors, particularly a PDE 2, 7 or 8 inhibitor, preferably a PDE2 inhibitor, said inhibitors preferably having an IC50 against the respective enzyme of less than 100nM;
12) one or more of a NPY (neuropeptide Y) inhibitor, more particularly NPY1 or NPY5 inhibitor, preferably NPY1 inhibitor, preferably said NPY inhibitors (including NPY Y1 and NPY Y5) having an IC50 of less than 100nM, more preferably less than 50nM; an assay for identifying NPY inhibitors is presented in WO-A-98/52890 (see page 96, lines 2 to 28);
13) one or more of vasoactive intestinal protein (VIP), VIP mimetic, VIP analogue, more particularly mediated by one or more of the VIP receptor subtypes VPAC1,VPAC or PACAP (pituitory adenylate cyclase activating peptide), one or more of a VIP receptor agonist or a VIP analogue (eg Ro-125-1553) or a VIP fragment, one or more of a α-adrenoceptor antagonist with VIP combination (eg invicorp, aviptadil);
14) one or more of a melanocortin receptor agonist or modulator or melanocortin enhancer, such as melanotan II, PT-14, PT-141 or compounds claimed in WO-09964002, WO-00074679, WO-09955679, WO-00105401, WO-00058361, WO-00114879, WO-00113112, WO-09954358;
15) one or more 5-HT₃ antagonist (preferably batanopride, granisetron, ondansetron, tropistron or MDL-73147EF);
16) one or more 5-HT₄ agonist (preferably cisapride and D-lysergic acid diethylamide);
17) one or more of testosterone, a testosterone replacement agent (inc dehydroandrostendione), testosternone (Tostrelle), dihydrotestosterone or a testosterone implant;
18) one or more of estradiol, estrogen, estrogen and medroxyprogesterone or medroxyprogesterone acetate (MPA) (i.e. as a combination), or estrogen and methyl testosterone hormone replacement therapy agent (e.g. HRT especially Premarin, Cenestin, Oestrofeminal, Equin, Estrace, Estrofem, Elleste Solo, Estring, Eastraderm TTS, Eastraderm Matrix, Dermestril, Premphase, Preempro, Prempak, Premique, Estratest, Estratest HS, Tibolone);
19) one or more of a modulator of transporters for noradrenaline, dopamine and/or serotonin, such as bupropion and GW-320659;
20) one or more of a purinergic receptor agonist and/or modulator;
21) one or more of a neurokinin (NK) receptor antagonist, including those described in WO-09964008;
22) one or more of an opioid receptor agonist, antagonist or modulator;
23) one or more of a modulator of cannabinoid receptors;
24) gabapentene;
25) one or more angiotensin-converting enzyme (ACE) inhibitors, e.g. quinapril; and
26) one or more anti-depressants; for example selective serotonin re-uptake inhibitors (sertraline, fluoxetine, fluvoxamine, paroxetine, citalopram, venlafaxine, mirtazapine, nefazodone, trazodone); tricyclic antidepressants (TCA, associated with cardiovascular side effects) clomipramine, desipramine, imipramine, amitriptyline, doxepin, amoxapine, maprotiline, nortriptyline, protriptyline, trimipramine, bupropion); and monoamine oxidase inhibitors phenelzine, tranylcypromine.

Preferred additional active agents for combination with the PDE5 inhibitors (preferably sildenafil, vardenafil, IC-351, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one and 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one) for use with the invention are selected from the list:
a) one or more α-adrenergic receptor antagonists (preferably prazosin, trazodone, alfuzosin, indoramin, tamsulosin, phenoxybenzamine, yohimbine, doxazosin, terazosin, clonidine, Recordati 15/2739, SNAP 1069, SNAP 5089 and RS17053);
b) one or more 5-HT₃ antagonist (preferably batanopride, granisetron, ondansetron, tropistron or MDL-73147EF);
c) one or more of a modulator of transporters for noradrenaline, dopamine and/or serotonin, such as bupropion and GW-320659; and
d) an anti-depressant (preferably sertraline, fluoxetine, fluvoxamine, paroxetine, citalopram, venlafaxine, clomipramine).

Particularly preferred additional active agents for combination with sildenafil for use with the invention are selected from the list: sertraline, fluoxetine, paroxetine, clomipramine, ondansetron, phenoxybenzamine, alfuzosin and terazosin.

Particularly preferred additional active agents for combination with 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one for use with the invention are selected from the list: sertraline, fluoxetine, paroxetine, clomipramine, ondansetron, phenoxybenzamine, alfuzosin and terazosin.

Particularly preferred additional active agents for combination with 5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one for use with the invention are selected from the list: sertraline, fluoxetine, paroxetine, clomipramine, ondansetron, phenoxybenzamine, alfuzosin and terazosin.

It is to be appreciated that all references herein to treatment include curative, palliative and prophylactic treatment.

If a combination of active agents are administered, then they may be administered simultaneously, separately or sequentially.

It will be appreciated that the invention provides basis for the following further aspects and that the embodiments specified hereinabove for the first aspect extend to these aspects:
i) a PDE5 inhibitor for treating premature ejaculation in patients with normal erectile function;
ii) a pharmaceutical combination for treating premature ejaculation in patients with normal erectile function comprising a PDE5 inhibitor and an additional active agent as hereinabove defined;
iii) the use of a pharmaceutical combination for the manufacture of a medicament for the treatment of premature ejaculation in patients with normal erectile function comprising a PDE5 inhibitor and an additional active agent as hereinabove defined;
iv) a kit for treating premature ejaculation in patients with normal erectile function, the kit comprising: a) a first pharmaceutical composition comprising a PDE5 inhibitor; b) a second composition comprising an additional active agent as hereinabove defined; and c) a container for the first and second compositions;
v) the use of a kit in the manufacture of a medicament for treating premature ejaculation in patients with normal erectile function, the kit comprising: a) a first pharmaceutical composition comprising a PDE5 inhibitor; b) a second composition comprising an additional active agent as hereinabove defined; and c) a container for the first and second compositions;
vi) a method of treating a patient suffering from premature ejaculation with normal erectile function comprising treating said patient with an effective amount of a PDE5 inhibitor; and
vii) a method of treating a patient suffering from premature ejaculation with normal erectile function comprising treating said patient with pharmaceutical combination comprising a PDE5 inhibitor and an additional active agent as hereinabove defined.

The following study was conducted to investigate the use of PDE5 inhibitors for the treatment of PE in patients with normal erectile function.

The study was conducted using sildenafil (Viagra®), however it will be appreciated that the study may be conducted with other PDE5 inhibitors, for example one or more of the preferred PDE5 inhibitors listed hereinabove.

The study comprised a phase II, placebo-controlled study to assess the efficacy of oral sildenafil (Viagra™) one hour prior to sexual intercourse in patients with premature ejaculation but with normal erectile function (i.e. patients scoring greater than 22 on the Erectile Function Domain Questionnaire (see hereinafter)) The following efficacy variables (end points) were used to evaluate the study.
i) The Intra-vaginal Ejaculatory Latency Time (IELT) - This formed the primary efficacy variable. The change in IELT from baseline of the Viagra group was compared to that of the placebo group. IELT was determined by stopwatch and was recorded by the patient via a diary. The patient recorded the IELT for each sexual event. Patients were asked to collect data on IELT for the first intra-vaginal penetration during any single event. In addition, the diary captured information on dosing and corresponding sexual intercourse attempts, and was completed when the patient had taken study medication and/or engaged in sexual activity.
ii) Index of Premature Ejaculation (IPE) - This index recorded the effects of the patient's sexual problems on his sex life: The index comprised a series of questions. This formed a secondary efficacy endpoint of the study.
iii) Sexual Quality of Life (Male) questionnaire (SQoL-M) - This questionnaire assesses the quality of sex life prior to and after treatment. This formed a secondary efficacy endpoint of the study.
iv) Global Efficacy Question (GEQ) - This question recorded the improvement in the overall level of satisfaction during sexual intercourse with the treatment. This formed a secondary efficacy endpoint of the study.
v) Time to ejaculation using penile vibratory stimulation - A number of patients were asked to record their time to ejaculation using penile vibratory stimulation. This technique is a reliable method for measuring ejaculation latency. Penile vibrators are available commercially, for example "FERTICARE® personal" (see www.multicept.com/ferticare.html, Multicept A/S, Lyngsø Alle 3, 2970 Horsholm, Denmark).

### Results

Preliminary results from the study demonstrate the efficacy of PDE5 inhibitors in treating PE in patients with normal erectile function.

### GEQ

Table 1 shows preliminary results from the GEQ. The results demonstrate a statistically significant improvement in the overall level of satisfaction during sexual intercourse in patients taking sildenafil compared to those taking placebo.

**Table 1:**

| GEQ: Has the treatment you have been taking improve your overall level of satisfaction during sexual intercourse? | | | |
|---|---|---|---|
| | Number of patients | Number that experienced an improvement | % that experienced an improvement |
| Placebo | 56 | 11 | 19.64 |
| Viagra | 72 | 27 | 37.50 |

### IPE

Preliminary results from the IPE demonstrated a statistically significant improvement in the patients' sexual problems in response to a number of questions. In particular, a statistically significant improvement was seen in the responses to the following questions with patients taking sildenafil compared to those taking placebo
- How often did you ejaculate before you were ready?
- How confident were you with your control over ejaculation?
- How satisfied were you with the quality of your orgasm?
- How satisfied have you been with your overall sex life?

In addition, an improvement was seen in the responses to the following questions with patients taking sildenafil compared to those taking placebo
- How many times did you engage in sexual intercourse?
- How often have you limited the amount of foreplay you engage in?
- How satisfied were you with your sense of control over your ejaculation?

### Components of the Erectile Function Domain Questionnaire.

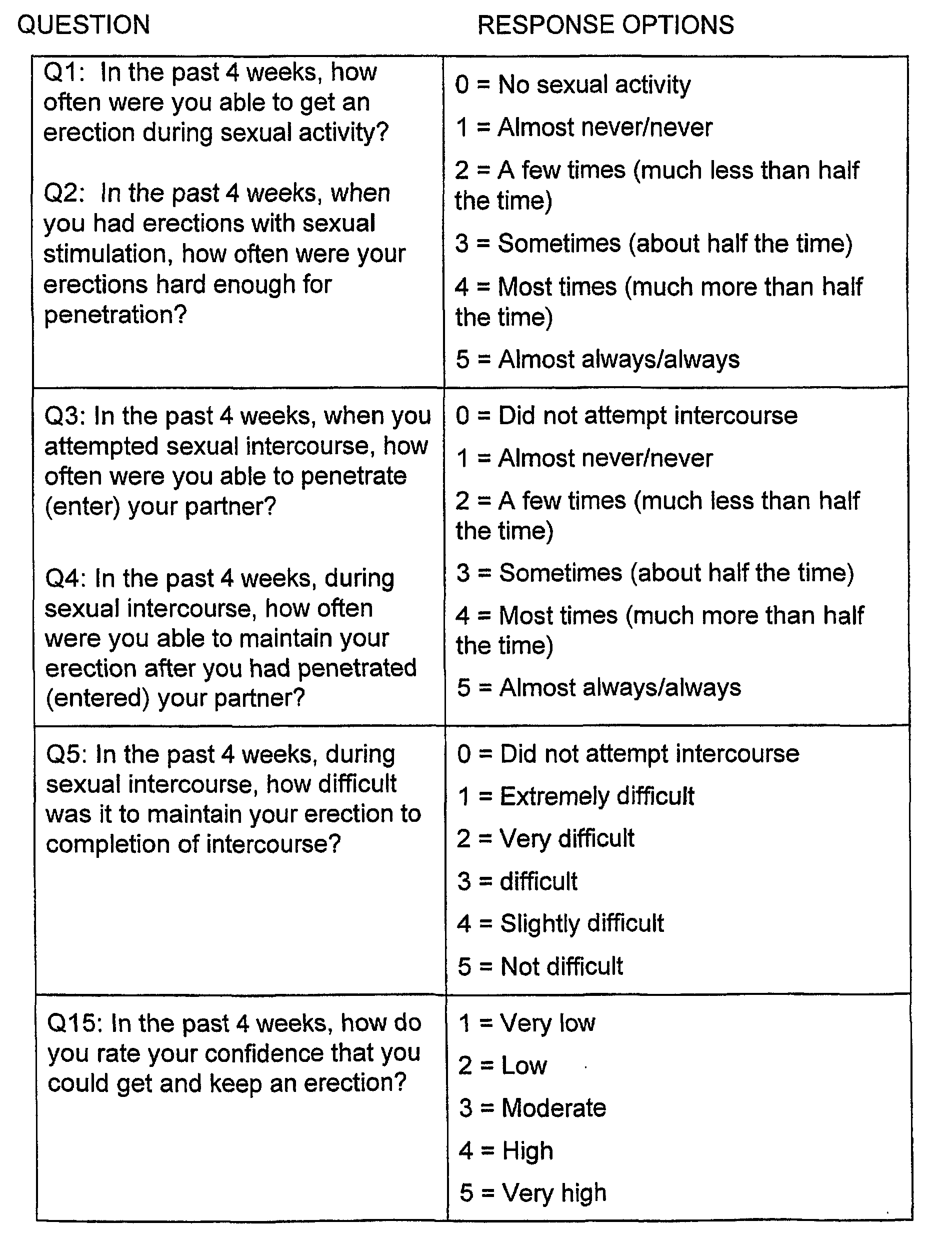

### Assay

PDE potency values referred to herein are determined by the following assays.

Preferred PDE compounds suitable for use in accordance with the present invention are potent and selective PDE5 inhibitors. *In vitro* PDE inhibitory activities against cyclic guanosine 3',5'-monophosphate (cGMP) and cyclic adenosine 3',5'-monophosphate (cAMP) phosphodiesterases can be determined by measurement of their IC₅₀ values (the concentration of compound required for 50% inhibition of enzyme activity).

The required PDE enzymes can be isolated from a variety of sources, including human corpus cavernosum, human and rabbit platelets, human cardiac ventricle, human skeletal muscle and bovine retina, essentially by the method of W.J. Thompson and M.M. Appleman (Biochem., 1971, 10, 311). In particular, the cGMP-specific PDE (PDE5) and the cGMP-inhibited cAMP PDE (PDE3) can be obtained from human corpus cavernosum tissue, human platelets or rabbit platelets; the cGMP-stimulated PDE (PDE2) was obtained from human corpus cavernosum; the calcium/calmodulin (Ca/CAM)-dependent PDE (PDE1) from human cardiac ventricle; the cAMP-specific PDE (PDE4) from human skeletal muscle; and the photoreceptor PDE (PDE6) from bovine retina. Phosphodiesterases 7-11 can be generated from full length human recombinant clones transfected into SF9 cells.

Assays can be performed either using a modification of the "batch" method of W.J. Thompson et al. (Biochem., 1979, 18, 5228) or using a scintillation proximity assay for the direct detection of AMP/GMP using a modification of the protocol described by Amersham plc under product code TRKQ709017100. In summary, the effect of PDE inhibitors was investigated by assaying a fixed amount of enzyme in the presence of varying inhibitor concentrations and low substrate, (cGMP or cAMP in a 3:1 ratio unlabelled to [³H]-labeled at a conc ∼1/3 *K*_{*m*}) such that IC₅₀ ≅ *K*_{*i*}. The final assay volume was made up to 100µl with assay buffer [20 mM Tris-HCl pH 7.4, 5 mM MgCl₂, 1 mg/ml bovine serum albumin]. Reactions were initiated with enzyme, incubated for 30-60 min at 30°C to give <30% substrate turnover and terminated with 50 µl yttrium silicate SPA beads (containing 3 mM of the respective unlabelled cyclic nucleotide for PDEs 9 and 11). Plates were re-sealed and shaken for 20 min, after which the beads were allowed to settle for 30 min in the dark and then counted on a TopCount plate reader (Packard, Meriden, CT) Radioactivity units were converted to % activity of an uninhibited control (100%), plotted against inhibitor concentration and inhibitor IC₅₀ values obtained using the 'Fit Curve' Microsoft Excel extension.

## Claims

1. The use of a PDE5 inhibitor for the manufacture of a medicament for treating premature ejaculation in patients with normal erectile function.

2. The use according to claim 1 wherein the patients attain a score of more than 22 on the Erectile Function Domain Questionnaire.

3. The use according to either claim 1 or claim 2 wherein the PDE5 inhibitor has an IC50 against the PDE5 enzyme of less than 100 nanomolar.

4. The use according to claim 3 wherein the PDE5 inhibitor has a selectivity over PDE3 of greater than 100 fold.

5. The use according to claim 4 wherein the PDE5 inhibitor has a selectivity over both PDE3 and PDE4 of greater than 100 fold.

6. The use according to claim 5 wherein the PDE5 inhibitor has an IC50 against PDE5 of less than 100 nM and a selectivity over PDE3 of greater than 100 fold.

7. The use according to claim 6 wherein the PDE5 inhibitor is selected from the group:
5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil),
(6R, 12aR)-2,3,6,7,12,12a-hexahydro-2-methyl-6-(3,4-methylenedioxyphenyl)-pyrazino[2',1':6,1]pyrido[3,4-b]indole-1,4-dione (IC-351);
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil),
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one; and
5-(5-acetyl-2-butoxy-3-pyridinyl)-3-ethyl-2-(1-ethyl-3-azetidinyl)-2,6-dihydro-7*H*-pyrazolo[4,3-*d*]pyrimidin-7-one and pharmaceutically acceptable salts thereof.

8. The use according to any preceding claim wherein the inhibitor is administered orally.

9. The use according to claim 8 wherein the daily dosage is 5 to 500 mg.

10. The use according to claim 9 wherein the daily dosage is 10 to 100 mg.

## Patentansprüche

1. Verwendung eines PDE5 Inhibitors zur Herstellung eines Arzneimittels für die Behandlung der verfrühten Ejakulation bei Patienten mit normaler erektiler Funktion.

2. Verwendung gemäß Anspruch 1, wobei die Patienten ein Ergebnis von mehr als 22 bezüglich des Fragebogens "Erectile Function Domain" erzielen.

3. Verwendung gemäß Anspruch 1 oder 2, wobei der PDE5 Inhibitor gegenüber dem PDE5 Enzym einen weniger als 100 nanomolaren IC₅₀-Wert aufweist.

4. Verwendung gemäß Anspruch 3, wobei der PDE5 Inhibitor gegenüber PDE3 eine mehr als 100 fache Selektivität aufweist.

5. Verwendung gemäß Anspruch 4, wobei der PDE5 Inhibitor sowohl gegenüber PDE3 als auch gegenüber PDE4 eine mehr als 100 fache Selektivität aufweist.

6. Verwendung gemäß Anspruch 5, wobei der PDE5 Inhibitor gegenüber PDE5 einen IC₅₀-Wert von weniger als 100nM und gegenüber PDE3 eine mehr als 100 fache Selektivität aufweist.

7. Verwendung gemäß Anspruch 6, wobei der PDE5 Inhibitor aus der Gruppe 5-[2-Ethoxy-5-(4-methyl-1-piperazinylsulfonyl)phenyl]-1-methyl-3-n-propyl-1,6-dihydro-7H-pyrazolo[4,3-d]pyrimidin-7-on (Sildenafil), (6R,12aR)-2,3,6,7,12,12a-Hexahydro-2-methyl-6-(3,4-methylendioxyphenyl)-pyrazino[2',1' : 6,1 ]pyrido[3,4-b]indol-1,4-dion (IC-351), 2-[2-Ethoxy-5-(4-ethy1-piperazin-1-y1-1-sulfonyl)-phenyl]-5-methyl-7-propyl-3H-imidazo[5,1-f][1,2,4]triazin-4-on (Vardenafil), 5-[2-Ethoxy-5-(4-ethylpiperazin-1-ylsulfonyl)pyridin-3-yl]-3-ethyl-2-[2-methoxyethyl]-2,6-dihydro-7H-pyrazolo[4,3-d)pyrimidin-7-on und 5-(5-Acetyl-2-butoxy-3-pyridinyl)-3-ethyl-2-(1-ethyl-3-azetidinyl)-2,6-dihydro-7H-pyrazolo[4,3-d]pyrimidin-7-on und deren pharmazeutisch akzeptablen Salzen ausgewählt ist.

8. Verwendung gemäß einem der vorhergehenden Ansprüche, wobei der Inhibitor oral verabreicht wird.

9. Verwendung gemäß Anspruch 8, wobei die tägliche Dosis 5 bis 500 mg beträgt.

10. Verwendung gemäß Anspruch 9, wobei die tägliche Dosis 10 bis 100 mg beträgt.

## Revendications

1. Utilisation d'un inhibiteur de PDE5 pour la fabrication d'un médicament pour le traitement de l'éjaculation prématurée chez des patients ayant une fonction érectile normale.

2. Utilisation selon la revendication 1, dans laquelle les patients atteignent une note supérieure à 22 au Questionnaire d'Evaluation de la Fonction Erectile.

3. Utilisation selon la revendication 1 ou 2, dans laquelle l'inhibiteur de PDE5 a une CI₅₀ vis-à-vis de l'enzyme PDE5 inférieure à 100 nanomoles.

4. Utilisation selon la revendication 3, dans laquelle l'inhibiteur de PDE5 a une sélectivité par rapport à la PDE3 supérieure à 100 fois.

5. Utilisation selon la revendication 4, dans laquelle l'inhibiteur de PDE5 a une sélectivité par rapport tant à la PDE3 qu'à la PDE4 supérieure à 100 fois.

6. Utilisation selon la revendication 5, dans laquelle l'inhibiteur de PDE5 a une CI₅₀ vis-à-vis de la PDE5 inférieure à 100 nM et une sélectivité par rapport à la PDE3 supérieure à 100 fois.

7. Utilisation selon la revendication 6, dans laquelle l'inhibiteur de PDE5 est sélectionné dans le groupe :
5-[2-éthoxy-5-(4-méthyl-1-pipérazinylsulfonyl) phényl]-1-méthyl-3-n-propyl-1,6-dihydro-7H-pyrazolo[4,3-d] pyrimidin-7-one (sildenafil) ;
(6R,12aR)-2,3,6,7,12,12a-hexahydro-2-méthyl-6-(3,4-méthylènedioxyphényl)-pyrazino[2',1':6,1]pyrido[3,4-b] indole-1,4-dione (IC 351) ;
2-[2-éthoxy-5-(4-éthylpipérazin-1-yl-1-sulfonyl)phényl]-5-méthyl-7-propyl-3H-imidazo[5,1-f][1,2,4]triazin-4-one (vardenafil) ;
5-[2-éthoxy-5-(4-éthylpipérazin-1-sulfonyl)pyridin-3-yl]-3-éthyl-2-[2-méthoxyéthyl]-2,6-dihydro-7H-pyrazolo[4,3-d]pyrimidin-7-one ; et
5-(5-acétyl-2-butoxy-3-pyridinyl)-3-éthyl-2-(1-éthyl-3-azétidinyl)-2,6-dihydro-7*H*-pyrazolo[4,3-*d*]pyrimidin-7-one et leurs sels pharmaceutiquement acceptables.

8. Utilisation selon l'une quelconque des revendications précédentes dans laquelle l'inhibiteur est administré par voie orale.

9. Utilisation selon la revendication 8, dans laquelle la dose quotidienne est de 5 à 500 mg.

10. Utilisation selon la revendication 9, dans laquelle la dose quotidienne est de 10 à 100 mg.
